Europäisches Patentamt

European Patent Office

Office européen des brevets

(19)

(11) Veröffentlichungsnummer: **0 394 841**

**A2**

(12) **EUROPÄISCHE PATENTANMELDUNG**

(21) Anmeldenummer: 90107436.9

(51) Int. Cl.⁵: **C07C 31/20, C07C 29/17**

(22) Anmeldetag: **19.04.90**

(30) Priorität: **27.04.89 DE 3913839**

(43) Veröffentlichungstag der Anmeldung:
**31.10.90 Patentblatt 90/44**

(84) Benannte Vertragsstaaten:
**BE CH DE FR GB IT LI NL**

(71) Anmelder: **BASF Aktiengesellschaft
Carl-Bosch-Strasse 38
D-6700 Ludwigshafen(DE)**

(72) Erfinder: **Toussaint, Herbert, Dr.
Knietschstrasse 11
D-6710 Frankenthal(DE)**
Erfinder: **Schossig, Juergen, Dr.
Raiffeisenstrasse 16
D-6701 Fussgoenheim(DE)**
Erfinder: **Graefje, Heinz, Dr.
Luxemburger Weg 2
D-6700 Ludwigshafen(DE)**
Erfinder: **Reiss, Wolfgang, Dr.
Bannwasserstrasse 70
D-6700 Ludwigshafen(DE)**
Erfinder: **Spahl, Roland, Dr.
Am Schanzenbuckel 19
D-6143 Lorsch(DE)**
Erfinder: **Irgang, Matthias, Dr.
Andreas-Hofer-Weg 41
D-6900 Heidelberg(DE)**
Erfinder: **Himmel, Walter, Dr.
Theodor-Storm-Strasse 12
D-6718 Gruenstadt(DE)**
Erfinder: **Koppenhoefer, Gerhard, Dr.
Nobelstrasse 16
D-6700 Ludwigshafen(DE)**

(54) Verfahren zur Hydrierung von Acetylenalkoholen.

(57) Verfahren zur Herstellung gesättigter Alkohole durch katalytische Hydrierung von Acetylenalkoholen bei Temperaturen von 50 bis 200°C und Drücken von 30 bis 320 bar, bei dem man einen Katalysator verwendet, der einen Gehalt von 20 bis 75 Gew.% Nickeloxid, 10 bis 75 Gew.% Zirkoniumdioxid und 5 bis 50 Gew.% Kupferoxid aufweist, wobei sich die Anteile jeweils auf den oxidischen nicht reduzierten Katalysator beziehen.

## Verfahren zur Hydrierung von Acetylenalkoholen

Diese Erfindung betrifft ein Verfahren zur Herstellung gesättigter Alkohole durch katalytische Hydrierung von Acetylenalkoholen.

Für die Hydrierung aliphatischer ungesättigter Verbindungen sind schon zahlreiche Katalysatoren vorgeschlagen worden. Beispielsweise ist aus der US-PS 3,449,445 bekannt, daß man Butin-2-diol-1,4 in guten Ausbeuten zu Butandiol-1,4 hydrieren kann, wenn man einen Katalysator verwendet, der Nickel, Kupfer und Mangan auf Siliciumdioxid enthält. Bei der großtechnischen Herstellung von Butandiol-1,4 nach diesem Verfahren treten jedoch Ablagerungen von Silicumdioxid in den Wärmeaustauschern und Rohrleitungen auf, welche sich nur durch sehr aufwendige Reinigungsoperationen entfernen lassen.

Mit dem in der DE-OS 25 36 276 beschriebenen trägerfreien Katalysator, der die Oxide von Nickel, Kupfer, Molybdän und Mangan enthält, werden bei der Hydrierung acetylenisch ungesättigter Alkohole gute Ergebnisse erzielt. Allerdings müssen bei der Hydrierung von Butin-2-diol-1,4 zu Butandiol-1,4, die bei einer Verschärfung der Reaktionsbedingungen (hohe Wasserstoffdrucke und hohe Temperaturen) erhaltenen besonders günstigen Resultate mit der unerwünschten Bildung von Butanol erkauft werden. Auch sind die in Strang- oder Tablettenform hergestellten Katalysatoren im großtechnischen Einsatz nicht formbeständig, so daß ein vorzeitiger Zerfall eintritt.

Nach den Angaben in der EP 0 018 569 werden mit einem Hydrierkatalysator, der die Oxide der Metalle Nickel, Kupfer, Molybdän und Mangan enthält, besonders vorteilhafte Ergebnisse bei der Hydrierung von Butin-2-diol-1,4 erzielt, wenn man bei der Herstellung des Katalysators durch Fällung der Metallsalze Filtrieren, Waschen, Trocknen und Tempern vor der Ausfällung ein Aluminium- oder Eisensalz zugibt. Beim Einsatz dieses Katalysators im großtechnischen Dauerbetrieb findet jedoch eine langsame, aber kontinuierliche Auflösung des Katalysators statt.

Es bestand deshalb die Aufgabe, ein neues Verfahren zur Hydrierung von Acetylenalkoholen zu entwickeln, das es ermöglicht, die ungesättigten Alkohole, wie Butin-2-diol-1,4, im technischen Dauerbetrieb in hohen Ausbeuten und unter Vermeidung der genannten Nachteile in die gesättigten Alkohole, wie Butandiol-1,4, zu überführen.

Nach dem Verfahren dieser Erfindung, das diese Anforderungen in hohem Maße erfüllt, stellt man gesättigte Alkohole durch katalytische Hydrierung von Acetylenalkoholen bei Temperaturen von 50 bis 200°C und Drücken von 30 bis 320 bar dadurch her, daß man einen Katalysator verwendet, der einen Gehalt an 20 bis 75 Gew.% Nickeloxid, 10 bis 75 Gew.% Zirkoniumdioxid und 5 bis 50 Gew.% Kupferoxid aufweist, wobei sich die Anteile jeweils auf den oxidischen nicht reduzierten Katalysator beziehen.

Zur Herstellung der gesättigten, insbesondere zweiwertigen Alkohole sind grundsätzlich alle Acetylenalkohole geeignet, die nach üblichen Verfahren erhalten werden können, wie Butin-2-diol-1,4 und Propargylalkohol.

Die katalytische Hydrierung der Acetylenalkohole wird mit Wasserstoff an dem Katalysator bei Temperaturen von 50 bis 200, vorzugsweise 80 bis 180°C und Drücken von 30 bis 320, vorzugsweise 100 bis 320 bar durchgeführt.

Der erfindungsgemäß verwendete Katalysator hat in seiner oxidischen, nicht reduzierten Form z.B. die folgende Zusammensetzung: 30 bis 70, vorzugsweise 40 bis 60, insbesondere 35 bis 55 Gew.% Nickeloxid, 10 bis 60, vorzugsweise 15 bis 50, insbesondere 25 bis 45 Gew.% Zirkoniumdioxid, 5 bis 40, vorzugsweise 10 bis 35, insbesondere 5 bis 20 Gew.% Kupferoxid. Der Katalysator kann außerdem z.B. 0,1 bis 5 Gew.% Molybdänoxid und gegebenenfalls z.B. 0 bis 10 Gew.% Manganoxid enthalten.

Ganz besonders gut ist das neue Verfahren für die Hydrierung von Butin-2-diol-1,4 zu Butandiol-1,4 geeignet. Bei dieser Hydrierung wird eine erhebliche Verlängerung der Katalysatorstandzeit und eine wesentliche Steigerung des Durchsatzes erzielt.

Die für das erfindungsgemäße Verfahren verwendeten Katalysatoren stellt man z.B. dadurch her, daß man Salze der Metalle Nickel, Kupfer, Zirkonium und gegebenenfalls Mangan auf an sich übliche Weise aus wäßriger Lösung bei einer Temperatur von 30 bis 90°C und einem pH-Wert von 5 bis 9 ausfällt, die Suspension filtriert und den Filterkuchen trocknet und bei einer Temperatur von 300 bis 700°C tempert, wobei man das Molybdän als Ammoniumheptamolybdat vor dem Trocknen zugibt. Dabei nimmt man die Fällung so vor, daß man eine wäßrige Lösung von Salzen, wie den Nitraten, Sulfaten oder Acetaten, der Metalle Nickel, Kupfer, Zirkonium und gegebenenfalls Mangan mit einer wäßrigen Lösung von Alkalicarbonat mischt. Die Menge der Metallsalze wird dabei so bemessen, daß die Katalysatormasse nach dem Tempern die angegebene Zusammensetzung aufweist.

Man kann das wasserlösliche Zirkoniumsalz auch teilweise, z.B. bis zu einem Anteil von 50 Gew.%, bezogen auf das eingesetzte Zirkonium, durch festes Zirkoniumdioxid ersetzen, welches man der wäßrigen Metallsalzlösung vor der Fällung zugibt oder im Reaktionsgefäß vorlegt.

Bei der Herstellung des Katalysators verfährt man im einzelnen z.B. so, daß man die wäßrige Lösung der Metallsalze gleichzeitig unter Rühren mit einer wäßrigen Alkalicarbonatlösung, vorzugsweise Natriumcarbonatlösung, vermischt, wobei die Metalle in Form eines Gemisches von Metallhydroxiden und Metallcarbonaten ausfallen. Der Metallsalzgehalt der Metallsalzlösung beträgt zweckmäßigerweise 30 bis 40 Gew.%. Die wäßrige Alkalicarbonatlösung ist z.B. 10 bis 20, vorzugsweise 15 bis 20 gew.%ig. Man fällt bei 30 bis 90, vorzugsweise bei 70 bis 90 °C und einem pH-Wert von 5 bis 9, vorzugsweise 7 bis 9.

Die erhaltene Suspension wird filtriert und so lange mit Wasser gewaschen, bis keine Anionen mehr nachgewiesen werden können. Dann wird z.B. bei einer Temperatur von 120 bis 200 °C im Trockenschrank oder einem Sprühtrockner getrocknet. Das Molybdän wird vorzugsweise als Ammoniumheptamolybdat dem feuchten Filterkuchen zugegeben. Der getrocknete Filterkuchen wird bei einer Temperatur von 350 bis 700 °C, vorzugsweise 400 bis 600 °C, getempert.

Zweckmäßigerweise wird die so erhaltene Katalysatormasse vor dem Einsatz auf an sich übliche Weise tablettiert oder extrudiert. Zum Beispiel verpreßt man die Katalysatormasse unter Verwendung eines Tablettierhilfsmittels, vorzugsweise Graphit, zu Tabletten mit den Dimensionen 6 x 3 mm. Die auf diese Weise hergestellten Tabletten werden bei einer Temperatur von 300 bis 700 °C, vorzugsweise 400 bis 600 °C, getempert. Die Tabletten haben ein Rüttelgewicht von 1 500 bis 1 900 g/l, eine Porisität (durch Wasseraufnahme bestimmt) von 0,2 bis 0,4 ml/g und eine Härte von 3 000 bis 4 000 $N/cm^2$. Der auf diese Weise erhältliche Katalysator wird vor seiner erfindungsgemäßen Verwendung einer reduktiven Behandlung mit Wasserstoff bei einer Temperatur von 200 bis 350 °C, vorzugsweise bei 230 bis 280 °C, beispielsweise über einen Zeitraum von 20 bis 40 Stunden bei Wasserstoffdrücken von 1 bis 300, vorzugsweise 100 bis 150 bar, unterworfen.

Die Hydrierung der genannten ungesättigten Verbindungen wird bei den angegebenen Temperaturen und Drücken z.B. in einem Reaktor durchgeführt, der den Katalysator in Form eines Festbettes enthält. Wie aus den folgenden Beispielen hervorgeht, werden hierbei erheblich längere Katalysatorstandzeiten und höhere Durchsätze als bei den bekannten Verfahren erzielt.

Als ein weiterer überraschender Vorteil des erfindungsgemäßen Verfahrens wurde gefunden, daß der Katalysator, der erst nach sehr langer Laufzeit desaktiviert wird, durch eine 8 bis 48 stündige Behandlung mit Wasser bei Temperaturen von 100 bis 250 °C, vorzugsweise 100 bis 200 °C und bei Drücken von 100 bis 320 bar nahezu vollständig reaktiviert werden kann.

Beispiele

Die in den Beispielen genannten Prozente sind Gewichtsprozente.

Beispiel 1

a) Herstellung des Katalysators

Eine wäßrige Lösung aus Nickelnitrat, Kupfernitrat und Zirkonacetat, die 4,48 % NiO, 1,52 % CuO und 2,82 % $ZrO_2$ enthält, wurde gleichzeitig in einem Rührgefäß in einem konstanten Strom mit einer 20 %igen wäßrigen Natriumcarbonatlösung bei einer Temperatur von 70 °C so gefällt, daß der mit einer Glaselektrode gemessene pH-Wert von 7,0 aufrechterhalten wurde.

Die erhaltene Suspension wurde filtriert und der Filterkuchen mit vollentsalztem Wasser gewaschen bis die elektrische Leitfähigkeit des Filtrates ca. 20 uS betrug. Dann wurde in den noch feuchten Filterkuchen so viel Ammoniumheptamolybdat eingearbeitet, daß das nachfolgend angegebene Oxidgemisch erhalten wurde. Danach wurde der Filterkuchen bei einer Temperatur von 150 °C in einem Trockenschrank oder einem Sprühtrockner getrocknet. Das auf diese Weise erhaltene Hydroxidcarbonatgemisch wurde nun bei einer Temperatur von 500 °C über einen Zeitraum von 4 Stunden getempert.

Der so erhaltene Katalysator hatte die Zusammensetzung: 50 % NiO, 17 % CuO, 1,5 % $MoO_3$ und 31,5 % $ZrO_2$. Das Katalysatorpulver wurde mit 3 Gew.% Graphit vermischt und zu 6 x 3-mm-Tabletten verformt. Die Tabletten hatten eine Porosität (gemessen über die Wasseraufnahme) von 0,20 ml/g und eine Härte von 3 500 $N/cm^2$.

b) Hydrierung von Butin-2-diol-1,4

Der nach Absatz a) erhaltene Katalysator wurde in einem Hydrierreaktor bei einer Temperatur von 250° C und einem Wasserstoffdruck von 150 bar reduziert. An 8 Volumenteilen des Katalysators wurden bei einer Temperatur von 150° C und einem Wasserstoffdruck von 250 bar 5 Gew.-Teile/h einer 50 %igen wäßrigen Lösung von Butin-2-diol-1,4 mit 2 500 Normvolumenteilen Wasserstoff hydriert. Zur Temperaturführung im Reaktor und zur Gewährleistung der erforderlichen Flüssigkeitsverteilung über das Katalysatorbett wurde der Butindiol-Zulauf mit 50 Volumenteilen/h Reaktoraustrag verdünnt. Die Hydrierwärme wurde über einen Wärmetauscher im Flüssigkeitskreislauf abgeführt. Nach Abtrennung der Gasphase von der Flüssigphase wurde das überschüssige Gas nach Ergänzung des verbrauchten Anteils durch Frischwasserstoff zum Reaktoreingang zurückgeführt.

Der Umsatz des Butin-2-diol-1,4 erfolgte nahezu vollständig. Nebenprodukte der Hydrierung (jeweils gerechnet wasserfrei) waren u.a.:

| | |
|---|---|
| Butanol | < 5 Gew.% |
| 2-Methylbutandiol-1,4 | < 0,2 Gew.% |
| Buten-2-diol-1,4 | < 0,1 Gew.% |
| [2-(4-Hydroxi)-butoxi]-oxalen | < 0,3 Gew.% |
| 4-Hydroxibutyraldehyd | < 0,5 Gew.% |
| gamma-Butyrolacton | < 0,5 Gew.% |

Zur weiteren Umsetzung der vier letztgenannten teilhydrierten Komponenten wurde der Austrag in einer zweiten Hydrierstufe im einfachen Durchgang am gleichen Katalysator bei Temperaturen von 180° C und einem Wasserstoffdruck von 250 bar nachhydriert.

Der anfallende Produktaustrag, der mindestens 94 Gew.% Butandiol-1,4 (berechnet wasserfrei) enthielt wurde destillativ zu Butandiol-1,4 rein aufgearbeitet. Auch nach einer Laufzeit von vier Monaten war noch kein deutlicher Druckverlust festzustellen. Weiterhin wurden keine Katalysatorbestandteile im Reaktoraustrag gefunden. Es wurden Butindiol-Durchsätze erzielt, welche um den Faktor drei höher lagen als beim folgenden Vergleichsversuch c).

c) Vergleichsversuch

Die in Absatz b) beschriebene Hydrierung wurde unter Verwendung des im Beispiel der EP-PS 18 569 beschriebenen Katalysators wiederholt. Schon nach einer Laufzeit von wenigen Wochen mußte die Katalysatorschüttung wegen Verstopfung ausgebaut werden.

Beispiel 2

Die in Beispiel 1, Absatz b) beschriebene Hydrierung wurde über einen längeren Zeitraum fortgesetzt. Nach einer Betriebszeit von ca. 3 Monaten wurde eine Abnahme der Hydrierleistung des Katalysators beobachtet, welche hauptsächlich durch Ablagerungen anorganischer Bestandteile aus der eingesetzten technischen Butindiollösung verursacht wurde. Das Katalysatorbett wurde 24 Stunden mit 200° C heißem Kondensat gespült. Nach dieser Behandlung wies der Katalysator wieder annähernd seine ursprüngliche Aktivität auf. Das Spülwasser enthielt neben Spuren an Zirkoniumdioxid u.a. 0,1 Gew.% Silicium, 0,03 Gew.% Natrium, 0,01 Gew.% Kupfer sowie organische Bestandteile (< 5 Gew.%).

**Ansprüche**

1. Verfahren zur Herstellung gesättigter Alkohole durch katalytische Hydrierung von Acetylenalkoholen bei Temperaturen von 50 bis 200° C und Drücken von 30 bis 320 bar, dadurch gekennzeichnet, daß man einen Katalysator verwendet, der einen Gehalt von 20 bis 75 Gew.% Nickeloxid, 10 bis 75 Gew.% Zirkoniumdioxid und 5 bis 50 Gew.% Kupferoxid aufweist, wobei sich die Anteile jeweils auf den oxidischen nicht reduzierten Katalysator beziehen.

4

2. Verfahren nach Anspruch 1, dadurch gekennzeichnet, daß man Butandiol-1,4 aus Butin-2-diol-1,4 herstellt.

3. Verfahren nach Anspruch 1, dadurch gekennzeichnet, daß man den Katalysator nach Abfall der Aktivität durch eine Behandlung mit Wasser bei Temperaturen von 100 bis 200°C und Drücken von 100 bis 320 bar reaktiviert.